# FASCICULE DE BREVET EUROPEEN

(11) **EP 3 435 994 B1**
(45) Date de publication et mention de la délivrance du brevet: **14.05.2025**
(21) Numéro de dépôt: 17717474.5
(22) Date de dépôt: 30.03.2017
(51) Int. Cl.: A61K 31/19, A61K 9/50, A61P 25/32

(54) **DOSES UNITAIRES À LIBÉRATION IMMÉDIATE DE GHB OU DE L'UN DE SES SELS THÉRAPEUTIQUEMENT ACCEPTABLES ADMINISTRÉES PAR VOIE ORALE ET LEUR UTILISATION POUR MAINTENIR L'ABSTINENCE ALCOOLIQUE**
EINHEITSDOSEN ZUR SOFORTFREISETZUNG VON HYDROXYBUTTERSÄURE ODER EINEM SEINER THERAPEUTISCH AKZEPTABLEN SALZE, ORAL VERABREICHT, UND IHRE VERWENDUNG ZUR AUFRECHTERHALTUNG DER ALKOHLABSTINENZ
UNITARY DOSES FOR IMMEDIATE DRUG RELEASE OF HYDROXY BUTANIC ACID OR OF ONE OF ITS THERAPEUTICALLY ACCEPTABLE SALTS ADMINISTERED ORALLY, AND THEIR USE FOR MAINTAINING ABSTINENCE FROM ALCOHOL

(30) Priorité: 01.04.2016 FR 1600554
(43) Date de publication de la demande: 06.02.2019
(73) Titulaire: Debregeas Et Associes Pharma, 75008 Paris (FR)
(72) Inventeur: GUIRAUD Julien, 921 10 CLICHY LA GARENNE (FR)
(74) Mandataire: Cabinet Netter
(86) Numéro de dépôt international: PCT/FR2017/000060
(87) Numéro de publication internationale: WO 2017/168059

(56) Documents cités:
- WO-A1-2011/119839
- WO-A1-2011/139271
- WO-A2-2006/053186
- US-A1- 2010 112 056
- US-B2- 8 729 070

## Description

La présente invention concerne des doses unitaires à libération immédiate de GHB ou de l'un de ses sels thérapeutiquement acceptables administrées par voie orale et de leur utilisation pour maintenir l'abstinence alcoolique.

L'oxybate de sodium, qui est le sel de l'acide hydroxybutyrique (GHB) le plus largement employé, est utilisé à des fins thérapeutiques dans le traitement des pathologies suivantes :
- dépendance à l'alcool : traitement du syndrome de sevrage alcoolique et maintien de l'abstinence alcoolique chez les patients alcoolo-dépendants
- narcolepsie chez les patients présentant une cataplexie,
- anesthésie.

Il est répertorié par la convention sur les substances psychotropes de 1971.

Dans le cadre d'une utilisation thérapeutique, il est important de bien déterminer la posologie optimale de l'oxybate de sodium de manière à favoriser le rapport bénéfice-risque du traitement et éviter tout risque de sur ou sous-dosage.

Le traitement de l'alcoolo-dépendance se déroule en deux phases.

Durant la première phase, les patients sont sevrés et éventuellement traités en cas de syndrome de sevrage sévère. Le syndrome du sevrage alcoolique est communément utilisé pour décrire le groupe de symptômes qui surviennent lors d'un arrêt progressif ou brutal d'alcool chez les patients alcoolo-dépendants. Ces manifestations traduisent un état de manque psychique, comportemental et physique. Le syndrome est, dans la majorité des cas, résolutif spontanément ou sous traitement en deux à cinq jours mais la mortalité n'est pas nulle (Société Française d'Alcoologie, 2006). Il est traité avec des anti-convulsants, des antipsychotiques ou avec de l'oxybate de sodium.

Après sevrage, une phase de maintien de l'abstinence est nécessaire afin d'améliorer l'état de santé du patient et d'éviter la survenance de pathologies liées à l'alcoolisme. L'abstinence semble être la seule méthode efficace pour faire cesser ou pour diminuer les atteintes psychologiques de l'alcoolisme. Elle est également le seul moyen de diminuer ses impacts sur la santé physique car il a été montré dans de nombreuses publications que les risques d'occurrence des morbidités et de la mortalité liées à l'alcool étaient fonction du niveau de consommation journalière d'alcool.

Le traitement du maintien de l'abstinence se compose de suivis psychosociaux et de traitements pharmacologiques sur des durées de plusieurs mois.

En Italie et en Autriche, l'oxybate de sodium est indiqué et utilisé dans le traitement du sevrage alcoolique et dans le maintien de l'abstinence depuis respectivement plus de 20 ans et 15 ans. Son efficacité dans ces indications et sa sécurité d'emploi ont été étudiées dans de nombreuses études cliniques publiées et le traitement a été administré à des centaines de milliers de patients.

Un article signé Giovanni Addolorato, Lorenzo Leggio, Anna Ferrulli, Fabio Caputo et Antonio Gasbarrini (Expert Opinion on Investigational Drugs. 2009 ; 18 (5) : 675-686) confirme que le GHB présente un intérêt dans le traitement des patients alcoolo-dépendants. Dans cet article les doses efficaces varient de 50 à 100 mg/kg et par jour (administrées en trois fois) en fonction de l'indication à savoir traitement du sevrage alcoolique ou maintien de l'abstinence.

La demande de brevet WO 2011/119839 décrit une composition pharmaceutique à libération contrôlée, et non pas à libération immédiate, alors que de dernier type de libération est indispensable dans le traitement de l'abstinence alcoolique. En outre, cette demande se réfère à l'utilisation du GHB dans le traitement de la narcolepsie et de la fibromyalgie, ce qui n'a rien à voir avec un traitement destiné au maintien de l'abstinence alcoolique.

La demande de brevet WO 2011/139271 décrit une composition pharmaceutique à libération immédiate, mais concerne le traitement de la narcolepsie.

La demande de brevet américain US 2010/112056 décrit également une composition pharmaceutique à libération immédiate, mais concerne le traitement de la narcolepsie, de la fibromyalgie et des troubles du sommeil.

La demande de brevet WO 2006/053186 décrit pour sa part une composition pharmaceutique destinée à traiter les mouvements anormaux.

Selon une étude plus récente de K. Skala et al., (Expert Opin. Pharmacoter. 2013 ; 15 (2)), la dose préconisée dans le domaine du maintien de l'abstinence alcoolique est de 50mg/kg répartie en trois prises par jour.

Sur la base de l'ensemble d'études cliniques, les autorités de santé autrichiennes et italiennes ont autorisé la prescription de l'oxybate de sodium dans le maintien de l'abstinence avec une posologie de 50 mg/kg/j répartie en trois prises pouvant être augmentée jusqu'à 100 mg/kg/j.

En matière de narcolepsie, un médicament utilisant l'oxybate de sodium comme principe actif et commercialisé sous la marque XYREM ^{®}, est préconisé avec des doses de 4,5 à 9 g par jour, réparties en deux prises par nuit. En outre divers brevets décrivent l'application du GHB dans le traitement de la narcolepsie, par exemple la demande internationale WO 2010/053691 ou la demande de brevet US 2014/0348917, toutes deux au nom de JAZZ Pharmaceuticals Inc.

De même, le brevet US 8591922 (Jazz Pharmaceuticals) décrit l'application du GHB dans le traitement de divers troubles du sommeil (notamment s'agissant d'apnées, de narcolepsies, de cataplexies, d'insomnies, de paralysies du sommeil).

Il est à noter que le brevet EP 1017381 (Orphan Medical Inc.) concerne des dérivés de butyrate, dont l'oxybate de sodium, utilisés dans le traitement de la fibromyalgie et du syndrome de fatigue chronique. Néanmoins, l'oxybate de sodium n'ayant pas démontré un rapport bénéfice-risque positif dans le traitement de la fibromyalgie et du syndrome de fatigue chronique, il ne dispose pas d'indication pour ces pathologies et il n'est donc en théorie pas utilisé à cet effet.

Le document US8729070 divulgue un traitement au GHB de la dépendance d'alcool sous forme de dosage oral de 50/mg/kg/jour.

Quelles que soient les indications retenues, il est connu que, pour des patients avec une insuffisance hépatique modérée ou sévère, les principaux paramètres pharmacocinétiques du GHB ou de l'un de ses sels sont augmentés d'un facteur pouvant aller jusqu'à plus de 120% pour une même dose administrée par rapport aux patients sans insuffisance hépatique (Ferrara et al., 1996). En conséquence, il est indiqué de diviser par deux les doses de GHB ou de ses sels pour ces patients par rapport à la posologie recommandée pour les patients sans insuffisance hépatique.

Selon la posologie et donc l'indication retenue, le GHB n'agit pas sur les mêmes récepteurs du système nerveux central en raison d'un dosage du principe actif différent ; ce dernier étant toujours calculé en fonction du poids du patient dans le cadre du traitement de l'alcoolo-dépendance.

Le GHB semble d'abord agir sur le récepteur GABA-B (acide gamma-aminobutyrique) mais il module directement ou indirectement l'activité d'autres neurotransmetteurs, notamment les systèmes dopaminergétiques, sérotoninergiques, opioïdes, cholinergiques, noradrénergiques, glutamatergiques (Pardi et al., 2006).

Le tableau ci-dessous résume ces différents paramètres en fonction de l'indication.

| | Alcoolo-dépendance | Narcolepsie | Anesthésie |
|---|---|---|---|
| Posologie | 50 à 100 mg/kg/j our | 4,5 à 9 g/jour | > 10 g |
| Mode d'administration | Par voie orale 3 prises/jour | Par voie orale 2 prises/nuit | Injection par voie intraveineuse |
| Récepteurs/neurotransmetteurs impliqués de façon prépondérante aux doses considérées | Dopaminergiques | GABA | GABA |
| Effets pharmaceutiques | Stimulant et anxiolytique, pouvant mimer les effets de l'alcool | Rétablissement de la qualité du sommeil paradoxal Sédatif | Adjuvant anesthésique |

Il ressort de ce tableau que l'effet du GHB va du simple effet stimulant jusqu'à la sédation et l'anesthésie, en fonction des doses utilisées.

Dans le cadre de la présente invention, le GHB trouve son application dans le traitement des troubles liés à l'alcool.

Plus précisément, l'application du GHB selon la présente invention se trouve être dans le maintien de l'abstinence en matière alcoolique.

Dans cette indication, l'art antérieur donne toujours des posologies pour le GHB exprimées en mg/kg, c'est-à-dire rapportées au poids du patient. Ces posologies signifieraient que la dose optimale est fonction du poids du patient. De surcroît, la littérature scientifique ne fournit aucune raison justifiant une dose rapportée au poids du patient, de sorte que le choix précis de la dose est essentiellement empirique, ce qui ne correspond pas forcément aux besoins du patient.

A l'encontre de cette pratique, la demanderesse a trouvé de façon tout à fait surprenante qu'il n'y avait aucune corrélation entre la dose efficace et le poids du patient et qu'une meilleure prise en charge des problèmes d'abstinence alcoolique était possible en ne faisant plus appel au poids du patient mais à sa consommation quotidienne en alcool, et ce indépendamment de son poids.

Dans une étude clinique de phase IIb/III récente portant sur 496 patients, quatre doses d'oxybate de sodium ont été comparées au placebo afin de vérifier que la posologie existante (50 mg/kg/j pouvant être augmenté jusqu'à 100 mg/kg/j) était optimale en termes d'efficacité, de sécurité et de tolérance. Les doses suivantes ont été étudiées :
- 0,75 g d'oxybate de sodium trois fois par jour - soit 2,25 g par jour (99 patients)
- 1,25 g d'oxybate de sodium trois fois par jour - soit 3,75 g par jour (99 patients)
- 1,75 g d'oxybate de sodium trois fois par jour - soit 5,25g par jour (99 patients)
- 2,25 g d'oxybate de sodium trois fois par jour - soit 6,75g par jour (100 patients)
- Groupe placebo (99 patients)

Les doses unitaires, conditionnées en sachet, étaient administrées par voie orale, sous forme solide, plus particulièrement sous forme de granulés.

L'efficacité était mesurée par le biais du pourcentage de jours abstinents (PDA), de la réduction de consommation d'alcool quotidienne (TAC) et par le nombre de jours de forte consommation (HDD). Il était prévu d'analyser la corrélation entre l'efficacité du traitement et la dose en mg/kg/j. A ce titre, la conversion en mg/kg/j a été effectuée en utilisant la formule suivante : dose unitaire en g d'oxybate de sodium x 3 / poids du patient.

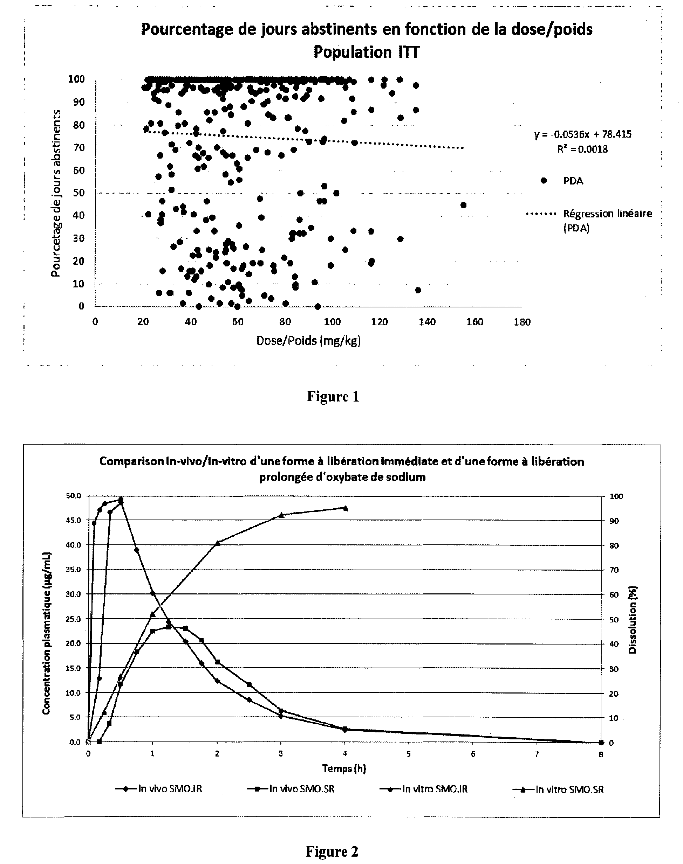

Les résultats sur l'ensemble de la population traitée avec l'oxybate de sodium (397 patients) sont rassemblés dans la **Figure 1** illustrant la relation efficacité vs dose en mg/kg.

Sur la base des résultats illustrés par cette Figure, il n'y a pas de relation entre l'efficacité du traitement exprimée en pourcentage de jours abstinents et le ratio dose/poids. En effet le coefficient de corrélation R² est proche de 0 (il est très précisément de 0,0014) montrant qu'il n'y a aucune corrélation entre l'efficacité et le ratio dose/poids ; quant à la pente de la droite, elle peut être considérée comme nulle (elle est non significativement négative).

Cette observation sur un nombre important de patients va à l'encontre de l'état de l'art.

Dans la suite de la présente description, il sera fait référence à deux catégories de patients alcooliques :
- les patients faiblement ou modérément alcooliques ;
- les patients fortement ou très fortement alcooliques.

Ces catégories sont fonction de la consommation quotidienne d'alcool de ces différents patients qui apparaît dans le tableau suivant :

| | **Consommation journalière d'alcool** | |
|---|---|---|
| Catégorie de patients | **Hommes** | **Femmes** |
| Faiblement alcooliques | 1 - 40 g / jour | 1 - 20 g / jour |
| Moyennement alcooliques | 41 - 60 g / jour | 21 - 40 g / jour |
| Fortement alcooliques | 61 - 100 g / jour | 41 - 60 g / jour |
| Très fortement alcooliques | > 101 g /jour | > 61 g /jour |

Ces valeurs correspondent aux différents niveaux de risques pour la santé du patient par rapport à une consommation d'alcool quotidienne - risque faible, modéré, élevé et très élevé - définis par l'OMS dans son document « WHO/MSD/MSB/00.4 ».

Bien que variant légèrement d'un pays à l'autre, un verre standard correspond sensiblement à 10-12 g d'alcool pur.

Dans le cadre de l'analyse des résultats de l'étude clinique de phase IIb/III présentée ci-dessus, la demanderesse a découvert de façon tout à fait surprenante que la dose optimale d'oxybate de sodium dans le maintien de l'abstinence dépendait du niveau de consommation d'alcool du patient avant le sevrage.

Les doses faibles (et non dépendantes du poids) étant les plus efficaces pour les patients faiblement ou modérément alcooliques et les doses plus élevées étant optimales pour les patients fortement ou très fortement alcooliques ; les doses optimales découvertes par la demanderesse se trouvent être significativement différentes de celles communément préconisées dans l'art antérieur, à savoir 50 à 100 mg/kg/jour répartis en trois prises. Par ailleurs, la demanderesse a pu montrer qu'un surdosage ou un sous-dosage par rapport à la posologie définie par la demanderesse pouvaient diminuer significativement l'efficacité du traitement et augmenter ses effets secondaires.

Ainsi, l'application de la posologie dont la demanderesse est à l'origine amène une amélioration de l'efficacité du traitement, la prévention d'un surdosage pouvant générer des effets secondaires indésirables et une simplification dans le traitement du patient alcoolique, dont on n'a plus à prendre en compte le poids pour ajuster la quantité de GHB ou de l'un de ses sels thérapeutiquement acceptables à lui délivrer quotidiennement.

La présente invention concerne donc des doses unitaires à libération immédiate contenant du GHB ou l'un de ses sels thérapeutiquement acceptables, tel que le sel de sodium, qui seront administrés aux patients trois fois par jour et chez qui on recherche un maintien de l'abstinence alcoolique. L'invention est définie dans les revendications annexées.

Dans la suite de la description le terme « GHB » couvrira à la fois l'acide gamma-hyroxybutyrique, ses sels pharmaceutiquement acceptables et en particulier son sel de sodium, dénommé oxybate de sodium.

La dose unitaire de GHB sera administrée sous forme orale solide, semi-solide, semi-liquide ou liquide et sera à libération immédiate telle que définie ci-après.

L'oxybate de sodium est un sel d'acide organique faible, l'acide gamma-hydroxybutyrique, ayant un pKa voisin de 4,5, et d'une base forte, l'hydroxyde de sodium. Il possède donc un pH à caractère basique et se trouve donc naturellement sous forme ionique au-dessus d'un pH de 4.5 environ. On ne connait pas de systèmes de transport actif pour le sodium oxybate. C'est donc sous sa forme non ionique, à un pH inferieur à son pKa (4,5) qu'il peut être absorbé par les muqueuses digestives. Ces conditions ne se retrouvent qu'au niveau de l'estomac.

Le temps moyen de demi-vidange gastrique (T50%) en condition « à jeun », c'est-à-dire 30 mn avant et 2 heures après les repas, est de 15 à 20 minutes. On attend donc d'une forme pharmaceutique délivrant un produit actif absorbé de façon significative uniquement en partie haute du tractus digestif, qu'elle libère ce produit dans un temps compatible avec celui de la vidange gastrique.

Dans le but de définir les formes à libération immédiate, le Département US de la Santé de la Food and Drug Administration décrit les caractéristiques physiques auxquelles elles doivent répondre dans un modèle de libération *in vitro* (Dissolution Testing of Immediate Release Solid Dosage Forms).

Par ailleurs, l'oxybate de sodium est classé dans le cas 1 des BCS (Biopharmaceutics Classification System) c'est-à-dire les produits hautement solubles et perméables, en l'occurrence plus particulièrement dans les conditions de pH précitées.

En toute logique, pour inclure une marge de sécurité, le Guide FDA recommande une norme de plus de 85% de produit actif libéré dans un milieu à pH 1 (HCl 0,1N), pH de l'estomac en conditions « à jeun », en 15 minutes pour respecter les contraintes de vidange gastrique.

S'agissant des granulés précités pris à titre d'exemple, ces derniers comprennent :
- le principe actif (GHB ou l'un de ses sels thérapeutiquement acceptables) ;
- un agent effervescent, tel que le bicarbonate de sodium ;
- un diluant, tel que l'aluminosilicate de magnésium, par exemple celui commercialisé sous la marque Neusilin ^{®} ;
- un liant, tel que la povidone ;
- un support, tel que celui constitué de sphères de sucre (sucrose mélangé à de l'amidon) ;
- un agent d'enrobage comprenant par exemple de l'hypromellose, de l'acide stéarique et du talc, éventuellement un agent aromatisant et un agent édulcorant ;

Les différents ingrédients, autres que le principe actif, à savoir l'agent effervescent, le diluant, le liant, le support, l'agent d'enrobage, l'agent aromatisant et l'agent édulcorant sont choisis parmi ceux qui sont cités dans la demande internationale WO 2012/107652 au nom de la demanderesse ; de même, le procédé d'obtention desdits granulés pourra être celui décrit dans ladite demande internationale.

Avantageusement, ces granulés ont la composition suivante (% par rapport au poids total du granulé) :
- Principe actif (oxybate de sodium) : 50 à 60% ;
- Agent effervescent : 5 à 15% ;
- Diluant : 2 à 18% ;
- Liant : 3 à 10% ;
- Support (cœur solide du granulé) : 15 à 25% ;
- Agent d'enrobage/ agent aromatisant / agent édulcorant / lubrifiant : 3 à 6%.

Plus spécifiquement, ces granulés ont la composition suivante (% par rapport au poids total du granulé) :
- Oxybate de sodium : 56,02% ;
- Bicarbonate de sodium : 8,40% ;
- Aluminosilicate de magnésium : 5,04% ;
- Povidone : 5,60% ;
- Sphères de sucre : 19,66% (à raison de 62,5% à 91,5% de sucrose pour 8,5% à 37,5% d'amidon) ;
- Agent d'enrobage : 0,95% d'hypromellose, 0,10% d'acide stéarique, 0,05% d'agent aromatisant, 0,83% de sucralose (agent édulcorant) et 3,34% de talc.

Avantageusement, ces granulés sont conditionnés en sachets, notamment en sticks, ce qui permettra une facilitation pour le praticien et pour le patient, évitant par exemple tout risque d'erreur quant à la posologie, tout en permettant un stockage et un transport plus faciles et plus sûrs.

Outre la forme « granulés » qui vient d'être décrite, d'autres formes pharmaceutiques (solides, semi-solides, semi-liquides ou liquides), toujours par voie orale et à libération immédiate, peuvent être envisagées sans pour autant sortir du cadre de la présente invention, sous réserve que ces formes présentent un profil pharmacocinétique semblable à celui desdits granulés.

A défaut de présenter un tel profil, la demanderesse a observé une chute de biodisponibilité du produit actif incompatible avec le maintien de l'efficacité.

Les tableaux qui suivent comparent les comportements *in vitro* et *in vivo* d'une forme à libération immédiate (SMO.IR) et d'une forme à libération non immédiate (SMO.SR). Cette étude a été réalisée chez 12 volontaires sains en cross-over.

Pour qu'une forme pharmaceutique de sodium oxybate soit considérée comme bioéquivalente selon le Guide EMA des Etudes de Bioéquivalence et soit susceptible de répondre aux caractéristiques d'efficacité, les paramètres pharmacocinétiques critiques (CMax et AUC) doivent être compris dans un intervalle de 80 à 125 % de ceux du produit de référence. Lorsque la valeur du TMax est critique, ce qui est le cas pour les formes immédiates à action rapide, ces valeurs ne doivent pas présenter de différence statistiquement significative.

| **Paramètres PK (Moyenne +/- Ecart Type)** | **SMO.IR Dose de 1,75 g d'oxybate de sodium** | **SMO.SR Dose de 1,75 g d'oxybate de sodium** |
|---|---|---|
| CMax (µg/mL) | 54.5 +/- 15.7 | 31.3 +/- 20.7 |
| TMax (h) | 0.50 | 1.25 |
| AUC t (µg/mL*h) | 70 +/- 46 | 52 +/- 39 |
| T ½ (h) | 0.56 +/- 0.26 | 0.56 +/- 0.26 |
| MRT | 1.20 +/- 0.47 | 1.78 +/- 0.45 |

T ½ : demi-vie d'élimination représentant le temps pour éliminer 50% des molécules absorbées.

Pour évaluer l'exposition des patients à deux formulations d'un même produit actif, il est préférable de comparer le MRT (Mean Residence Time) qui représente le temps de résidence moyen dans l'organisme d'une molécule d'oxybate de sodium et qui en l'occurrence tient compte de la cinétique d'absorption.

Malgré des T½ d'élimination logiquement équivalents, le MRT de la forme SR est significativement augmenté et provient d'une absorption plus lente.

Le paramètre le plus important dans la présente évaluation (développement d'une forme solide à libération immédiate) est le TMax. Le TMax de la forme immédiate (30 minutes) est cohérent avec les temps moyens de vidange gastrique et permet d'obtenir une AUC et un CMax optimum (pas de perte de produit) et donc une exposition du patient maitrisée. Une forme qui ne serait pas à libération immédiate (SMO.SR) induirait une chute de l'exposition (AUC et CMax significativement diminuées) et une perte d'efficacité.

La demanderesse a trouvé les valeurs suivantes de AUC et de CMax, qui dépendent des doses utilisées pour des doses de 0,32 g, 0,75 g, 1,75 g et 1,9 g :

| | 0,32 g | | 0,75 g | | 1,75 g | | 1,9 g | |
|---|---|---|---|---|---|---|---|---|
| | Moyenne | Ecart Type | Moyenne | Ecart Type | Moyenne | Ecart Type | Moyenne | Ecart Type |
| CMax (µg/mL) | 10 | 2,9 | 23 | 6,7 | 54,5 | 15,7 | 59,2 | 17 |
| AUCt (µg/mL*h) | 13 | 8 | 30 | 19,7 | 70 | 46 | 76 | 50 |

Pour répondre aux caractéristiques de l'invention, la forme pharmaceutique doit présenter idéalement un TMax de 30 minutes environ et en tous cas pas supérieur à 40 minutes (2 fois le temps maximal d'une demi-vie de vidange gastrique à jeun, soit 20 minutes).

La **Figure 2** illustre la comparaison *in vivo - in vitro* d'une forme à libération immédiate et d'une forme à libération non immédiate d'oxybate de sodium ; il ressort de cette Figure que les cinétiques de libération *in vitro* et d'absorption *in vivo* présentent la même pente. Le modèle de dissolution *in vitro* est donc prédictif du comportement PK des formulations.

Dans ce contexte, les formes pharmaceutiques devront présenter idéalement une libération *in vitro* du produit actif supérieure à 90% en 15 minutes et en tous cas supérieure à 85 % pour répondre aux définitions des formes immédiates en vigueur (Guides FDA du développement des formes à libération immédiate).

L'analyse des données de l'étude de phase IIb/III précitée a montré une interaction statistiquement très significative (p = 0,0012) entre l'efficacité du traitement, le niveau de consommation d'alcool journalier et la dose unitaire d'oxybate de sodium utilisée trois fois par jour.

Pour les deux catégories de patients analysées (cf. catégories décrites ci-dessus), des modèles linéaires et quadratiques sur le PDA ont montré des relations statistiquement significatives entre le niveau d'efficacité et la dose d'oxybate de sodium administrée aux patients.

### • Patients fortement ou très fortement alcooliques

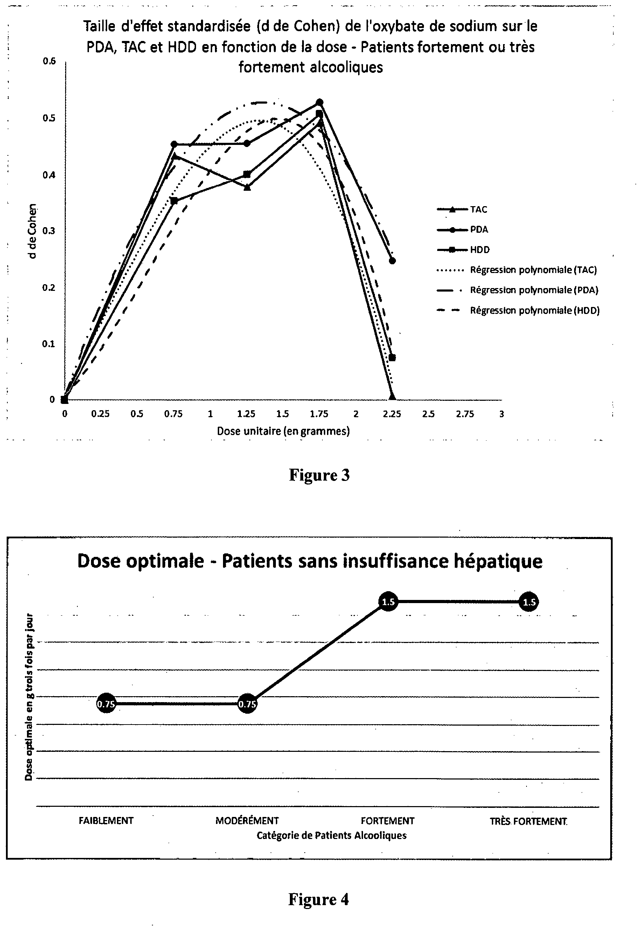

Chez les patients fortement ou très fortement alcooliques sans insuffisance hépatique, ces modèles statistiques ont montré que l'efficacité de l'oxybate de sodium dans le maintien de l'abstinence augmentait progressivement lorsque que les doses unitaires administrées trois fois par jour augmentaient, avec un résultat statistiquement significatif atteint avec la dose de 1,75 g (p < 0,05). C'est ce qui est illustré à la **Figure 3****.** Les modèles de régression polynomiale ont montré également que l'optimum chez les patients fortement ou très fortement alcooliques sans insuffisance hépatique est atteint avec la dose de 1,5g environ. Au-delà des doses de 1,75g ou en dessous de 1,25g, l'efficacité diminue sensiblement.

Ainsi, les doses comprises entre 1,25 g et 1,75 g d'oxybate de sodium trois fois par jour ont les résultats d'efficacité les plus élevés. La dose de 1,50 g trois fois par jour peut être considérée comme la dose optimale.

Il est important de souligner que les doses inférieures à 1,25 g ou supérieures à 1,75 g ont montré des résultats d'efficacité jusqu'à 80% inférieurs à la dose optimale.

### • Patients faiblement ou modérément alcooliques

Chez les patients faiblement ou modérément alcooliques sans insuffisance hépatique, les modèles statistiques ont montré un optimum de l'efficacité atteint avec la dose de 0,75 g trois fois par jour, puis une décroissance de l'efficacité pour les doses au-delà de 1,25 g d'oxybate de sodium trois fois par jour.

Ainsi, chez les patients faiblement ou modérément alcooliques, les doses unitaires comprises entre 0,75 g et 1,25 g d'oxybate de sodium trois fois par jour ont les résultats d'efficacité les plus élevés et la dose unitaire de 0,75 g trois fois par jour représente la dose optimale.

Les doses supérieures à 1,25 g ont montré une baisse d'efficacité pouvant aller jusqu'à environ 20% de l'efficacité de la dose optimale.

### • Relation Dose-Réponse

Comme cela a été précédemment mentionné, la dose unitaire selon l'invention est déterminée en fonction du niveau d'alcoolisme du patient.

La **Figure 4** représente la dose-réponse identifiée en fonction de la consommation d'alcool avant sevrage pour des patients sans insuffisance hépatique.

Pour les patients faiblement ou modérément alcooliques sans insuffisance hépatique, la dose de 0,75 g trois fois par jour apparait comme la dose optimale.

Pour les patients fortement ou très fortement alcooliques sans insuffisance hépatique, la dose de 1,50 g trois fois par jour apparait comme la dose optimale.

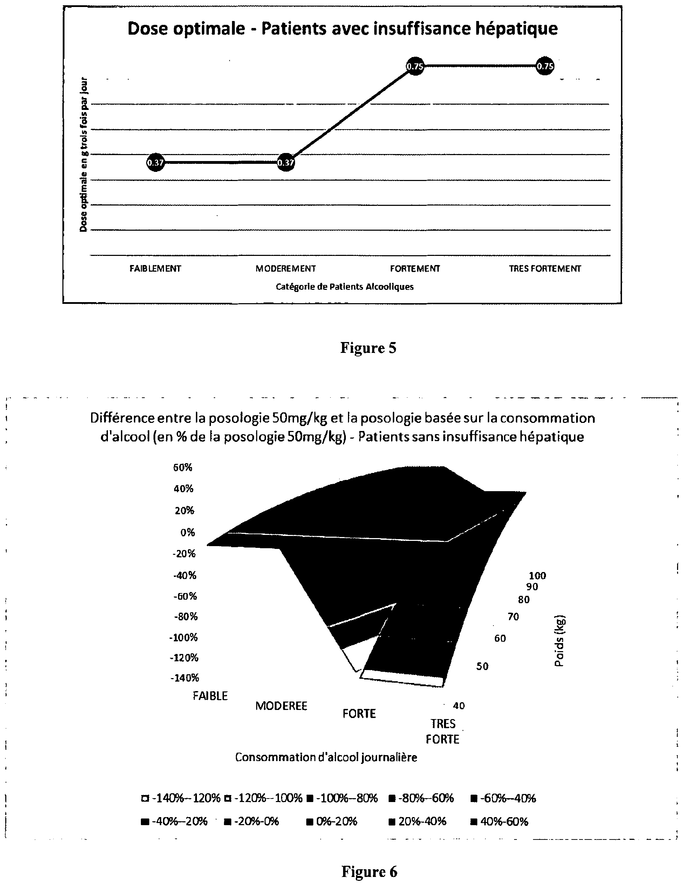

Comme indiqué précédemment, il convient de diviser par deux les doses pour les patients atteints d'insuffisance hépatique. Ainsi, pour ces patients, les fourchettes de doses pour les patients faiblement à modérément alcooliques se situent entre 0,37 et 0,62 g avec trois prises quotidiennes, et entre 0,62 et 0,87 g avec trois prises quotidiennes pour les patients fortement ou très fortement alcooliques. Les doses optimales à l'intérieur de ces fourchettes, soit 0,37 g pour les patients faiblement ou modérément alcooliques et 0,75 g pour les patients fortement ou très fortement alcooliques en trois prises quotidiennes sont illustrées à la **Figure 5****.**

Comme cela a été mentionné précédemment, la demanderesse a été à l'encontre de l'état de la technique qui préconise systématiquement un dosage prenant en compte le poids du patient.

Par ailleurs, il a été démontré qu'un mauvais dosage par rapport à la posologie optimale définie par la demanderesse pouvait entrainer une perte significative d'efficacité avec une augmentation des effets secondaires liés à l'oxybate de sodium.

La demanderesse a par ailleurs illustré dans les Figures ci-dessous que ces situations de délivrance de posologie non optimale peuvent être très fréquentes pour des patients sans insuffisance hépatique (Figures 6 et 7) ou avec insuffisance hépatique (Figures 8 et 9) :
Patients sans insuffisance hépatique :
   - la **Figure 6** représente la différence (en %) entre la posologie basée sur l'état de l'art (50 mg/kg/jour) et la posologie découverte par la demanderesse pour des patients sans insuffisance hépatique. Cette Figure indique que pour un patient faiblement ou modérément alcoolique pesant 75 kg sans insuffisance hépatique, la borne inférieure de la posologie telle que définie dans l'état de l'art (50 mg/kg/jour) aurait entrainé un surdosage de l'ordre de 40% par rapport à la posologie optimale découverte et établie par la demanderesse. A l'opposé et pour les patients fortement ou très fortement alcooliques sans insuffisance hépatique, la posologie telle que définie par l'état de l'art à 50 mg/kg/jour reviendrait à un sous-dosage pouvant aller jusqu'à -125% en fonction du poids du patients par rapport à la posologie optimale définie par la demanderesse.

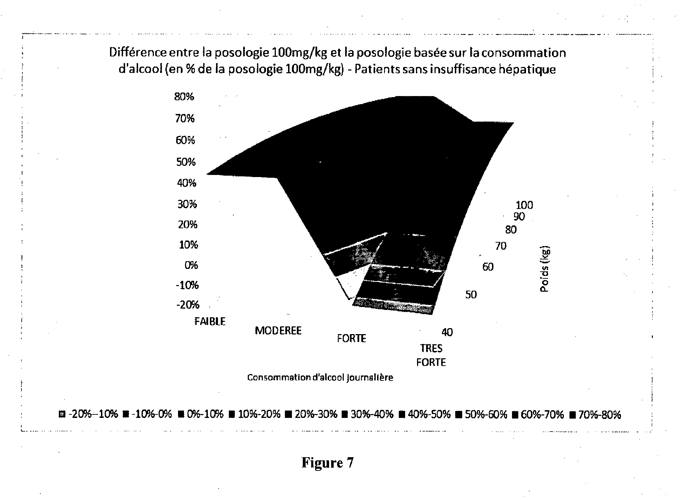

   - la **Figure 7** présente les différences (exprimées en %) entre la borne supérieure de la posologie provenant de l'état de l'art (100 mg/kg/jour) et la posologie découverte par la demanderesse. Cette Figure indique que pour un patient faiblement ou modérément alcoolique sans insuffisance hépatique, la borne supérieure de la posologie telle que définie dans l'état de l'art (100 mg/kg/jour) aurait entrainé un surdosage de l'ordre de 44% à 80% par rapport à la posologie optimale découverte et établie par la demanderesse. A l'opposé et pour les patients fortement ou très fortement alcooliques sans insuffisance hépatique, la posologie telle que définie par l'état de l'art à 100 mg/kg/jour reviendrait à un surdosage compris entre 10% et 55% pour les patients de plus de 50 kg par rapport à la posologie optimale définie par la demanderesse.
Patients avec insuffisance hépatique :

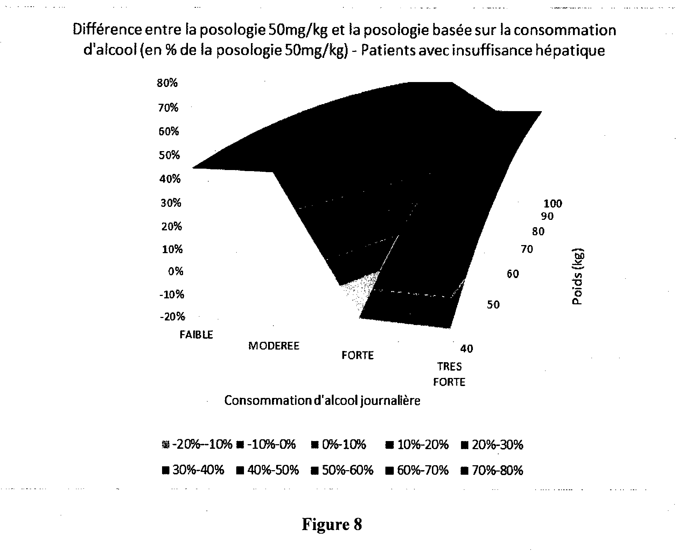

   - la **Figure 8** représente la différence (en %) entre la posologie basée sur l'état de l'art (50 mg/kg/jour) et la posologie découverte par la demanderesse pour des patients avec insuffisance hépatique. Cette Figure indique que pour un patient faiblement ou modérément alcoolique avec une insuffisance hépatique, la borne inférieure de la posologie telle que définie dans l'état de l'art (50 mg/kg/jour) aurait entrainé un surdosage de l'ordre de 45% à 80% par rapport à la posologie optimale découverte et établie par la demanderesse. A l'opposé et pour les patients fortement ou très fortement alcooliques avec une insuffisance hépatique, la posologie telle que définie par l'état de l'art à 50 mg/kg/jour reviendrait à un surdosage compris entre 10% et 55% pour les patients de plus de 50 kg par rapport à la posologie optimale définie par la demanderesse.

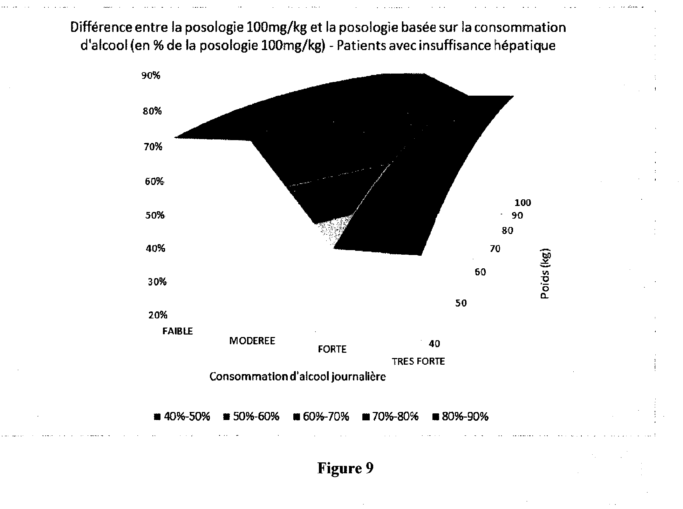

   - la **Figure 9** présente les différences (exprimées en %) entre la borne supérieure de la posologie provenant de l'état de l'art (100 mg/kg/jour) et la posologie découverte par la demanderesse pour un patient avec une insuffisance hépatique. Cette Figure indique que pour un patient faiblement ou modérément alcoolique avec insuffisance hépatique, la borne supérieure de la posologie telle que définie dans l'état de l'art (100 mg/kg/jour) aurait entrainé un surdosage de l'ordre de 72% à 89% par rapport à la posologie optimale découverte et établie par la demanderesse. Pour les patients fortement ou très fortement alcooliques avec une insuffisance hépatique, la posologie telle que définie par l'état de l'art à 100 mg/kg/jour reviendrait à un surdosage compris entre 44% et 78% par rapport à la posologie optimale définie par la demanderesse.

Il ressort de ce qui précède que les doses unitaires selon l'invention varient de 0,37 g à 1,75 g en trois prises par jours, des fourchettes plus restreintes et des doses optimales ayant par ailleurs été déterminées, tenant compte à la fois du degré d'alcoolémie du patient (faiblement à modérément alcoolique d'une part, ou fortement à très fortement alcoolique d'autre part), et du fait que ce patient présente on non une insuffisance hépatique.

En résumé, la demanderesse a pu déterminer que pour les patients faiblement ou modérément alcooliques sans insuffisance hépatique, une dose unitaire de 0,75 à 1,25 g, trois fois par jour donnait d'excellents résultats. En dessous de 0,75 g il est apparu que les résultats n'étaient pas suffisamment probants. Par ailleurs, les doses supérieures à 1,25 g trois fois par jour ont montré une efficacité significativement moindre pour des patients faiblement ou modérément alcooliques. Les meilleurs résultats ont été obtenus pour une dose unitaire de 0,75 g, avec trois prises quotidiennes.

Pour les patients faiblement ou modérément alcooliques avec une insuffisance hépatique, une fourchette de doses unitaires allant de 0,37 à 0,62 g avec trois prises quotidiennes a donné des résultats satisfaisants, étant entendu que la dose de 0,37 g trois fois par jour apparait comme la dose optimale.

S'agissant des patients fortement ou très fortement alcooliques sans insuffisance hépatique, la demanderesse a pu démontrer qu'une dose unitaire de 1,25 à 1,75 g avec trois prises par jour donnait les meilleurs résultats ; en dessous de 1,25 g, la dose n'est pas suffisante pour les patients fortement ou très fortement alcooliques et s'adresse plutôt, comme cela vient d'être mentionné, aux patients faiblement ou modérément alcooliques. Au-dessus de 1,75 g, l'efficacité décroit significativement et les effets secondaires deviennent plus nombreux et plus fréquents, en particulier un effet de sédation avec des nausées et une fatigue prononcée pour le patient ; la demanderesse a pu démontrer que la dose optimale pour les patients fortement ou très fortement alcooliques sans insuffisance hépatique était de 1,50 g avec trois prises par jour.

Pour les patients avec ce niveau de consommation d'alcool et une insuffisance hépatique, il est préconisé des doses allant de 0,62 à 0,87 g avec trois prises par jour, la dose optimale étant de 0,75 g trois fois par jour.

Finalement, ce n'est qu'exceptionnellement qu'un patient pour lequel on utiliserait la méthode classique (posologie déterminée en fonction du poids) aurait la dose appropriée telle que celle préconisée par la demanderesse ;

A ce titre la demanderesse a pu montrer que la méthode classique pouvait engendrer des sous-dosages ou des surdosages importants, pouvant aller jusqu'à plus de 10 fois la dose optimale déterminée par la demanderesse.

## Revendications

1. Dose unitaire de GHB ou de l'un de ses sels thérapeutiquement acceptables pour utilisation dans le maintien de l'abstinence alcoolique chez les patients souffrant d'alcoolisme, **caractérisée en ce que** la libération *in vitro* du principe actif est supérieure à 85% en quinze minutes dans un milieu à pH 1 (HCl 0,1N), et préférentiellement à 90% en quinze minutes, ladite dose unitaire étant choisie parmi
- une dose comprise entre 1,25 et 1,75 g, de préférence 1,50 g, pour l'utilisation trois fois par jour dans le maintien de l'abstinence alcoolique chez les patients fortement ou très fortement alcooliques sans insuffisance hépatique tel que défini par l'OMS dans WHO/MSD/MSB/00.4 ;
- une dose comprise entre 0,75 et 1,25 g, de préférence 0,75 g, pour l'utilisation trois fois par jour dans le maintien de l'abstinence alcoolique chez les patients faiblement ou modérément alcooliques sans insuffisance hépatique tel que défini par l'OMS dans WHO/MSD/MSB/00.4 ;
- une dose comprise entre 0,62 et 0,87 g, de préférence 0,75 g, pour l'utilisation trois fois par jour dans le maintien de l'abstinence alcoolique chez les patients fortement ou très fortement alcooliques avec une insuffisance hépatique tel que défini par l'OMS dans WHO/MSD/MSB/00.4 ; et
- une dose comprise entre 0,37 et 0,62 g, de préférence 0,37 g, pour l'utilisation trois fois par jour dans le maintien de l'abstinence alcoolique chez les patients faiblement ou modérément alcooliques avec une insuffisance hépatique tel que défini par l'OMS dans WHO/MSD/MSB/00.4.

2. Dose unitaire pour l'utilisation selon la revendication 1, **caractérisée en ce qu'**elle se présente sous forme orale solide, semi-solide, semi-liquide ou liquide, ces différentes formes présentant le même profil pharmacocinétique.

3. Dose unitaire pour l'utilisation selon la revendication 2, **caractérisée en ce que** ladite forme orale présente un TMax inférieur à 40 mn, et préférentiellement inférieur à 30 minutes.

4. Dose unitaire pour l'utilisation selon l'une des revendications 2 ou 3, **caractérisée en ce que** ladite forme orale solide est constituée de granulés.

5. Dose unitaire pour l'utilisation selon la revendication 4, **caractérisée en ce que** lesdits granulés sont conditionnés en sachets, notamment en sticks.

6. Dose unitaire pour l'utilisation selon l'une des revendications précédentes dans laquelle ledit principe actif est l'oxybate de sodium.

7. Dose unitaire pour l'utilisation selon l'une des revendications 4 ou 5, **caractérisée en ce que** lesdits granulés ont la composition suivante (donnée par rapport au poids des granulés) :
∘ principe actif : 50 à 60% ;
∘ agent effervescent : 5 à 15% ;
∘ diluant : 2 à 18% ;
∘ liant : 3 à 10% ;
∘ support (cœur solide du granulé) : 15 à 25% ;
∘ agent d'enrobage/ agent aromatisant / agent édulcorant / lubrifiant : 3 à 6%.

8. Dose unitaire pour l'utilisation selon la revendication 7, **caractérisée en ce que** lesdits granulés ont la composition suivante (données par rapport au poids des granulés):
∘ Oxybate de sodium (principe actif) : 56,02% ;
∘ Bicarbonate de sodium (agent effervescent) : 8,40% ;
∘ Aluminosilicate de magnésium (diluant) : 5,04% ;
∘ Povidone (liant) : 5,60% ;
∘ Sphères de sucre (support) : 19,66% (à raison de 62,5% à 91,5% de saccharose pour 8,5% à 37,5% d'amidon) ;
∘ Agent d'enrobage : 0,95% d'hypromellose, 0,10% d'acide stéarique, 0,05% d'agent aromatisant, 0,83% de sucralose (agent édulcorant) et 3,34% de talc.

## Patentansprüche

1. Einheitsdosis von GHB oder eines seiner therapeutisch akzeptablen Salze zur Verwendung zur Aufrechterhaltung der Alkoholabstinenz bei Patienten mit Alkoholismus, **dadurch gekennzeichnet, dass** die *In-vitro-*Freisetzung des Wirkstoffs in einem Medium mit pH 1 (HC1 0,1 N) in fünfzehn Minuten mehr als 85 % und vorzugsweise 90 % in fünfzehn Minuten beträgt, wobei die Einheitsdosis ausgewählt ist aus
- einer Dosis zwischen 1,25 und 1,75 **g,** vorzugsweise 1,50 g, für die Anwendung dreimal täglich zur Aufrechterhaltung der Alkoholabstinenz bei Patienten mit starkem oder sehr starkem Alkoholkonsum ohne Leberfunktionsstörung gemäß der Definition der WHO in WHO/MSD/MSB/00. 4;
- einer Dosis zwischen 0,75 und 1,25 g, vorzugsweise 0,75 g, für die Anwendung dreimal täglich zur Aufrechterhaltung der Alkoholabstinenz bei Patienten mit geringem oder mäßigem Alkoholkonsum ohne Leberfunktionsstörung gemäß der Definition der WHO in WHO/MSD/MSB/00. 4;
- einer Dosis zwischen 0,62 und 0,87 g, vorzugsweise 0,75 g für die Anwendung dreimal täglich zur Aufrechterhaltung der Alkoholabstinenz bei Patienten mit starkem oder sehr starkem Alkoholkonsum mit Leberfunktionsstörung gemäß der Definition der WHO in WHO/MSD/MSB/00.4; und
- einer Dosis zwischen 0,37 und 0,62 g, vorzugsweise 0,37 g, zur Anwendung dreimal täglich zur Aufrechterhaltung der Alkoholabstinenz bei Patienten mit niedrigem oder mäßigem Alkoholkonsum mit Leberfunktionsstörung gemäß der Definition der WHO in WHO/MSD/MSB/00.4.

2. Einheitsdosis für die Verwendung nach Anspruch 1, **dadurch gekennzeichnet, dass** sie in oraler fester, halbfester, halbflüssiger oder flüssiger Form vorliegt, wobei diese verschiedenen Formen das gleiche pharmakokinetische Profil aufweisen.

3. Einheitsdosis für die Verwendung nach Anspruch 2, **dadurch gekennzeichnet, dass** die orale Form eine TMax von weniger als 40 Minuten und vorzugsweise weniger als 30 Minuten aufweist.

4. Einheitsdosis zur Verwendung nach einem der Ansprüche 2 oder 3, **dadurch gekennzeichnet, dass** die feste orale Form aus Granulaten besteht.

5. Einheitsdosis für die Verwendung nach Anspruch 4, **dadurch gekennzeichnet, dass** das Granulat in Beuteln, insbesondere in sticks, verpackt ist.

6. Einheitsdosis zur Verwendung nach einem der vorhergehenden Ansprüche, wobei der Wirkstoff Natriumoxybat ist.

7. Einheitsdosis für die Verwendung nach einem der Ansprüche 4 oder 5, **dadurch gekennzeichnet, dass** die Granulate folgende Zusammensetzung aufweisen (bezogen auf das Gewicht der Granulate):
∘ Wirkstoff: 50 bis 60 %;
∘ Brausemittel: 5 bis 15 %;
∘ Verdünnung: 2 bis 18 %;
∘ Bindemittel: 3 bis 10 %;
∘ Trägermaterial (fester Kern des Granulats): 15 bis 25 %;
∘ Einbettmittel/ Aromamittel/ Süßungsmittel/ Schmiermittel: 3 bis 6 %.

8. Einheitsdosis für die Verwendung nach Anspruch 7, **dadurch gekennzeichnet, dass** das Granulat folgende Zusammensetzung aufweist (Angaben in Bezug auf das Gewicht des Granulats):
∘ Natriumoxybat (Wirkstoff): 56,02 %;
∘ Natriumbicarbonat (Brausemittel): 8,40 %;
∘ Magnesium-Aluminiumsilikat (Verdünnung): 5,04 %;
∘ Povidon (Bindemittel): 5,60 %;
∘ Zuckerkugeln (Träger): 19,66 % (im Verhältnis von 62,5 % bis 91,5 % Saccharose zu 8,5 % bis 37,5 % Stärke):
∘ Einbettmittel: 0,95 % Hypromellose, 0,10 % Stearinsäure, 0,05 % Aroma, 0,83 % Sucralose (Süßungsmittel) und 3,34 % Talkum.

## Claims

1. Unit dose of GHB or of one of therapeutically acceptable salts thereof for use in the maintaining of alcohol abstinence in patients with an alcohol dependence, **characterized in that** the *in vitro* release of the active ingredient is more than 85% in fifteen minutes in a medium with pH 1 (HCl 0,1N), and preferably than 90% in fifteen minutes, said unit dose being selected from
- a dose comprised between 1,25 and 1,75 g, preferably 1,50 g for use three times per day in the maintaining of alcohol abstinence in patients with severe or very severe alcohol dependence without a liver failure as defined by WHO in WHO/MSD/MSB/00.4;
- a dose comprised between 0,75 and 1,25 g, preferably 0,75 g, for use three times per day in the maintaining of alcohol abstinence in patients with mild or moderate alcohol dependence without a liver failure as defined by WHO in WHO/MSD/MSB/00.4;
- a dose comprised between 0,62 and 0,87 g, preferably 0,75 g, for use three times per day in the maintaining of alcohol abstinence in patients with severe or very severe alcohol dependence with a liver failure as defined by WHO in WHO/MSD/MSB/00.4; and
a dose comprised between 0,37 and 0,62 g, preferably 0,37 g, for use three times per day in the maintaining of alcohol abstinence in patients with mild or moderate alcohol dependence with a liver failure as defined by WHO in WHO/MSD/MSB/00.4.

2. Unit dose for use according to claim 1, **characterized in that** it is in a solid, semisolid, semi-liquid or liquid oral form, those different forms having the same pharmacokinetic profile.

3. Unit dose for use according to claim 2, **characterized in that** said oral form has a TMax less than 40 mn, and preferably less than 30 minutes.

4. Unit dose for use according to one of claims 2 or 3, **characterized in that** said solid oral form is composed of granules.

5. Unit dose for use according to claim 4, **characterized in that** said granules are packaged in sachets, in particulars in sticks.

6. Unit dose for use according to one of the preceding claims wherein said active ingredient is sodium oxybate.

7. Unit dose for use according to one of claims 4 or 5, **characterized in that** said granules have the following composition (given in relation to the weight of the granules):
∘ active ingredient: 50 to 60%;
∘ effervescent agent: 5 à 15%;
∘ diluent: 2 to 18%;
∘ binder: 3 to 10%;
∘ substrate (solid core of the granule): 15 à 25%;
∘ coating agent/flavouring agent/sweetening agent/ lubricant: 3 to 6%;

8. Unit dose for use according claim 7, **characterized in that** said granules have the following composition (given in relation to the weight of the granules):
∘ Sodium oxybate (active ingredient): 56,02%;
∘ Sodium bicarbonate (effervescent agent): 8,40%
∘ Magnesium aluminosilicate (diluent): 5,04%;
∘ Povidone (binder): 5,60%;
∘ Sugar spheres (substrate): 19,66% (in a proportion of 62,5% to 91,5% sucrose per 8,5% to 37,5% starch);
∘ Coating agent: 0,95% hypromellose, 0,10% stearic acid, 0,05% flavouring agent, 0,83% sucralose (sweetening agent) and 3,34% talc.
